# EUROPEAN PATENT SPECIFICATION

(11) **EP 0 532 512 B1**
(45) Date of publication and mention of the grant of the patent: **11.12.1996**
(21) Application number: 91907987.1
(22) Date of filing: 25.03.1991
(51) Int. Cl.: A61K 31/55, A61K 31/44, A61K 31/365, A61K 45/06

(54) **USE OF PLATELET ACTIVATING FACTOR ANTAGONISTS AS ANTI-PRURITIC AGENTS**
VERWENDUNG VON ANTAGONISTEN DES PLÄTTCHEN-AKTIVIERENDEN FAKTORS BEI PRURITUS
EMPLOI D'ANTAGONISTES DE FACTEUR D'ACTIVATION PLAQUETTAIRE COMME AGENTS ANTIPRURITIQUES

(30) Priority: 31.05.1990 US 530739
(43) Date of publication of application: 24.03.1993
(73) Proprietor: ALLERGAN, INC., Irvine, California 92713-9534 (US)
(72) Inventor: WOODWARD, David, Frederick, El Toro, CA 92630 (US); WILLIAMS, Linda, Sue, Santa Ana, CA 92701 (US)
(74) Representative: Griffin, Kenneth David
(86) International application number: US9102003
(87) International publication number: WO9118608

(56) References cited:
- EP-A- 0 194 416
- US-A- 4 734 280
- THE JOURNAL OF PHARMACOLOGY AND EXPERIMENTAL THERAPEUTICS vol. 242, no. 1, July 1987, pages 263 - 268 TERASHITA Z.I. ET AL 'CV-6209, A HIGHLY POTENT ANTAGONIST OF PLATELET ACTIVATING FACTOR IN VITRO AND IN VIVO.'
- ACTA DERMATO-VENEREOLOGICA vol. 65, no. 5, 1985, STOCKHOLM pages 409 - 12 FJELLNER B. ET AL 'EXPERIMENTAL PRURITUS EVOKED BY PLATELET ACTIVATING FACTOR (PAF-ACETHER) IN HUMAN SKIN'
- BRITISH JOURNAL OF PHARMACOLOGY vol. 97, no. 1, May 1989, pages 171 - 180 HELLEWELL P.G. ET AL 'ANTAGONISM OF PAF-INDUCED OEDEMA FORMATION IN RABBIT SKIN A COMPARISON OF DIFFERENT ANTAGONISTS'
- BRITISH JOURNAL OF DERMATOLOGY vol. 122, no. 4, April 1990, pages 539 - 544 KEMENY L. ET AL 'EFFECT OF BN 52021, A PLATELET ACTIVATING FACTOR ANTAGONIST, ON DITHRANOL-INDUCED INFLAMMATION'
- THE NEW ENGLAND JOURNAL OF MEDICINE vol. 313, no. 7, 15 August 1985, pages 405
- - 409 GRANDEL KAREN E. ET AL 'ASSOCIATION OF PLATELET-ACTIVATING FACTOR WITH PRIMARY ACQUIRED COLD URTICARIA'

## Description

### Field of the Invention

This invention concerns the use of platelet activating factor (PAF) antagonists or anti-pruritic agents.

### Background of the Invention

Itch, or pruritus, is a common and distressing symptom in a variety of diseases. Pruritus typically occurs in peripheral diseases such as allergic conjunctivitis, allergic rhinitis, hemorrhoids, and dermatoses of fungal, allergic and non-allergic origin. Itching can also be a major symptom of certain systemic diseases such as, Hodgkin's disease, chronic renal failure, polycythema vera, hyperthyroidism and cholestasis [see, for example, Herndon, J.H., Jr., Int. J. Derm. 14, 465-484 (1975); Winkelmann, R.K., Med. Clins. N. Am. 66, 1119-1133 (1982)]. The clinical importance of pruritus is undeniable but research efforts in this area have been modest, to great extent due to the absence of established, specific experimental models, especially in preclinical research.

The intracutaneous injection of histamine or proteases elicits itch, and may be used as an experimental model for itch studies [Cormia, F.E., J. Invest. Derm. 19, 21 (1952); Shelley, W.B. and Arthur, R.P., Arch. Derm. (Chicago) 76, 296, 323 (1957)]. It was, therefore, postulated that these agents are involved as mediators in various itching conditions. Since histamine was believed to be the primary mediator of the itch sensation, conventional itch therapy involves H₁-antihistamines as a first-line medication. However, antihistamines have no general anti-pruritic effect, in many instances they are either ineffective or only partially effective. The physician is often obliged to resort to glucocorticoids to relieve pruritus but the potential undesirable side effects from glucocorticoid therapy are of great concern. Glucocorticoids cause skin atrophy and are absorbed systemically to cause Cushings disease-like effects. It has been concluded that although histamine is undoubtedly a potent pruritogen, at least one other itch-producing substance is involved in the clinically encountered spectrum of diseases where itch is a major symptom.

Although it is known that experimental pruritus may be evoked in human skin by the local administration of diverse pharmacologically active substances, the majority of which cause inflammation, demonstration that a chemical substance causes an itch sensation when locally administered does not necessarily mean that it is involved (as a mediator) in diseases in which itching is a symptom. Substances which have been reported to evoke or facilitate the itch sensation in human skin have not led to accepted anti-itch medications in those instances where compositions of matter are available to block the synthesis or activity of such substances. For example, according to Hagermark et al., J. Invest. Dermatol. 69, 527-539 (1977), prostaglandins E₂ and H₂ produce itch in human skin and potentiate the itch evoked by histamine. However, according to an earlier article by Hagermark [Acta Dermatovener 53, 363-368 (1973)] a known inhibitor of PGE₂ synthesis, aspirin, did not act as an anti-pruritic agent, rather it actually prolonged experimental itch produced by trypsin or histamine. These experimental findings are amply supported by clinical experience where drugs like aspirin and indomethacin are not generally regarded as useful in treating itch.

The pruritogenic activity of other substances has been attributed to an indirect mechanism involving histamine release, these postulates being based on the activity of systemically administered or locally injected H₁-antihistamines. Thus, synthetic platelet activating factor (PAF) has been reported to cause pruritus in human skin [Fjellner and Hagermark, Acta Derm. Venereol. 65, 409-412 (1985)] "via indirect and mainly histamine-dependent mechanism". Based upon their experimental findings, the authors concluded that PAF probably produced itch in human skin by release of mast cell bound histamine.

Given the complicating factors which may confound interpretation of itching studies, proof of involvement of a substance in mediating itch is provided only by studies which demonstrate that a composition of matter which interferes which the synthesis or pharmacological action of the substance in question attenuates pruritus in either an experimental model of itching disease or in a study involving clinically encountered itch. Further, the beneficial effect of such a composition of matter in relieving itch should be demonstrated as independent of the actions of histamine.

### Summary of the Invention

The present invention is based on the unexpected finding that platelet activating factor (PAF) is a very potent pruritogen and platelet activating factor antagonists (PAF antagonists) of highly diverse structures prevent itching episodes of allergic origin. The previously unrecognized anti-pruritic activity of such PAF antagonists is demonstrated as being independent of histaminergic mechanisms.

In another aspect, the invention relates to the use of PAF antagonists in the preparation of pharmaceutical compositions intended for the treatment of pruritus.

The PAF antagonists may, for example, be selected from synthetic PAF analogues, natural products isolated from plants having PAF antagonist activity, and triazolobenzodiazepines. The PAF antagonists are preferably applied topically to the afflicted site but systemic such as oral, parenteral, nasal and intrarectal administration, is also possible.

### Detailed Description of the Invention

Platelet activating factor (PAF) is a term coined by Benveniste et al. [J. Exp. Med. 136, 1356-1377 (1972)) to describe a fluid phase mediator of unknown chemical structure. This mediator has later been identified as a phospholipid autocoid, which structurally is 1-O-alkyl-2-acetyl-sn-glycero-3-phosphocholine of the formula (I) 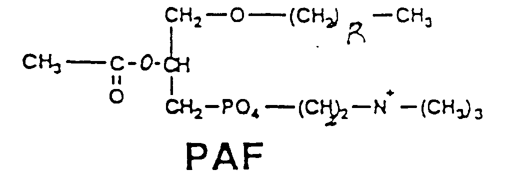 wherein R is 15 or 17, i.e. the alkyl moiety is hexadecyl or octadecyl. The isolation, chemical synthesis and biological and biochemical characteristics of PAF are, for example, disclosed by Hanahan and Kumar in Prog. Lipid Res. 26, 1-28 (1987).

PAF is not stored in cells but is synthesized in response to appropriate stimuli by a two-step process. The precursor 1-O-alkyl-(R)acyl-glycero-3-phosphatidylcholine is converted to lyso-PAF by phospholipase A₂ and subsequent acetylation results in PAF formation. In fact lyso-PAF is both a precursor and a metabolite of PAF. PAF is known to be an important factor of physiological reactions including platelet aggregation, inflammation, contraction of smooth muscle, alterations in the respiratory and circulatory systems, etc.

PAF antagonists have been discovered comparatively recently, and comprise a series of compounds of diverse structures effective in the treatment of conditions traditionally associated with PAF. The PAF antagonists reported so far in the art may be broadly classified according to their origin and structures as follows: (a) synthetic analogues of the PAF structure; (b) natural products isolated from plants; (c) triazolobenzodiazepines. By virtue of these antagonists, it has become apparent that PAF exerts its known biological effects by stimulating specific PAF-sensitive receptors.

A PAF antagonist, which is a synthetic analogue of the PAF structure is the compound of formula (II), 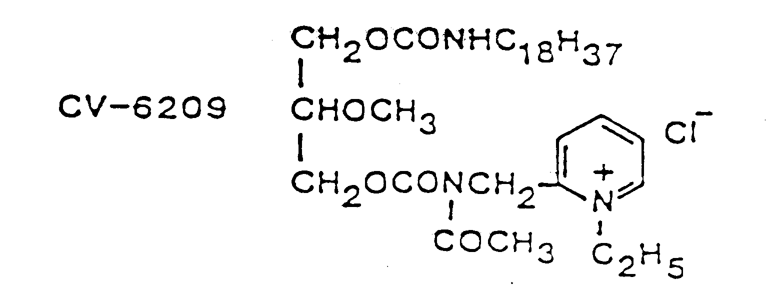 which was disclosed by Terashita et al., In J. Pharm. Exp. Ther. 242, 263-268 (1987). Other synthetic lipid PAF inhibitors are described In the Published European Patent Application 0,157,609 A2 as preventive or therapeutic agents for a variety of circulatory diseases and allergic disorders and as anti-neoplastic agents.

According to the United States Patent No 4,734,280, PAF-induced maladies can be effectively treated by the administration of a ginkgolide or a ginkgolide derivative. Ginkgolides were originally isolated from Ginkgo Biloba extracts, and their commonly available representatives include Ginkgolide A, Ginkgolide B, Ginkgolide C, and Ginkgolide M, of which Ginkgolide B (BN 52021) of the formula (III) 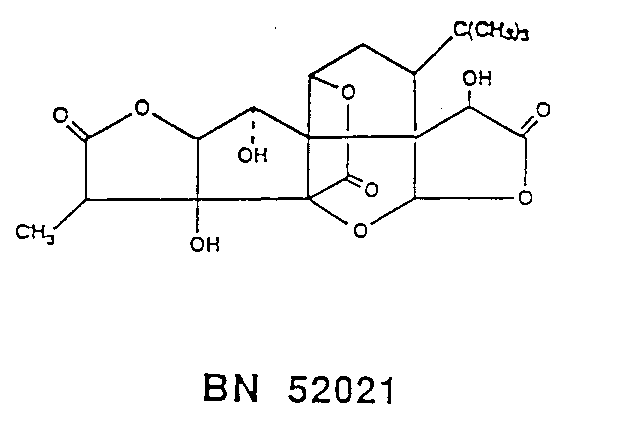 was found to be the most effective [Braquet et al., L. Actualites de Chimie Therapeutique (Paris) 13, 237-254 (1986)]. Known derivatives of ginkgolides include the mono-acetate, the tri-acetate, and the tetrahydro and acetyl derivatives. According to the test data disclosed in the U.S. Patent No. 4,734,280, these compounds show platelet aggregation inhibiting and antianaphylactic activities, and exhibit a protective effect against transvascular fluid escape and shock.

PAF antagonist triazolobenzodiazepine derivatives were, for example, described by Kornecki et al., Science 226, 1454-1456 (1986), and are disclosed in the United States Patent No. 4,820,703 , and in the Published European Patent Application No. 0,194,416 A1. A typical representative of this class of PAF antagonists is the compound of formula (IV) 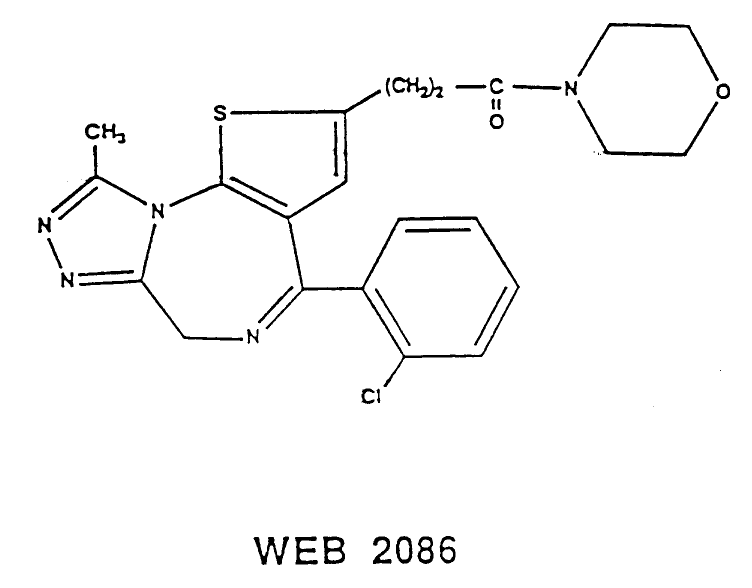 (WEB 286) which is disclosed in the above-cited Published European Patent Application. These compounds are described as useful for the prevention or treatment of various PAF-induced diseases, such as diseases associated with platelet aggregation, certain immediate allergic reactions caused by PAF, pain, edema, alteration in the respiratory and circulatory system, etc.

In studies intended to identify pruritogenic substances other than histamine, the inventor of the present application has discovered that PAF is a most potent itch producing substance. Moreover, certain known PAF antagonists were found to specifically block the pruritogen activity of PAF and, more importantly, were found to significantly reduce the pruritus associated with an experimental model of itching diseases. Although PAF had been described as a substance that causes pruritus in human skin by Fiellner and Hagermark, Supra, its action was associated with histamine liberation. Accordingly, it was entirely unexpected that PAF antagonists are capable of relieving itch, and do so in a non-histamine related fashion.

The term "PAF" antagonist as used in accordance with the present invention, refers to synthetic or naturally occurring compounds known in the art or hereinafter discovered, that are effective in the treatment of pruritus via interfering with the pruritogenic action of PAF. This definition includes, but is not restricted to the above-mentioned three classes of PAF antagonists, the preferred representatives being compounds of formulae (II), (III) and (IV) as hereinabove defined.

The term "treatment" is used to cover all aspects of the control of itching including prophylaxis and therapy.

The term "therapeutically effective amount" and grammatical variations thereof, as used herein refer to sufficient quantities of the active compound that can produce the desired therapeutic effect when administered to a mammal afflicted with pruritus. The term "therapeutic effect" is used herein in a broad sense and includes prophylactic effects.

In accordance with the present invention, the PAF antagonists are preferably applied to the afflicted area topically, in admixture with pharmaceutical carriers, in the form of topical pharmaceutical compositions. Such compositions include solutions, suspensions, lotions, gels, creams, ointments, emulsions, skin patches, etc. All of these dosage forms, along with methods for their preparation, are well known in the pharmaceutical and cosmetic art. Typically, such topical formulations contain the active ingredient in a concentration range of 0.001 to 10 mg/ml, in admixture with suitable vehicles. Other desirable ingredients for use in such anti-pruritic preparations include preservatives, co-solvents, viscosity building agents, carriers, etc.

For ophthalmic application, preferably solutions are prepared typically containing from about 0.001 to about 10 mg/ml, preferably from about 0.1 to about 6 mg/ml of active ingredient, and a physiological saline solution as a major vehicle. The pH of such ophthalmic solutions should preferably be maintained between 6.5 and 7.2 with an appropriate buffer system. The formulations may also contain conventional, pharmaceutically acceptable preservatives, stabilizers and/or penetration enhancers.

The preferred vehicle that may be used in the ophthalmic solutions of the present invention is purified water, more preferably a physiological saline solution. Additional suitable vehicles include but are not restricted to, viscosity agents such as polyvinyl alcohol, povidone, hydroxypropyl methyl cellulose, poloxamers, carboxymethyl cellulose, carbomer and hydroxyethyl cellulose.

Preferred preservatives that may be used in the ophthalmic formulations of the present invention include, but are not limited to, benzalkonium chloride, chlorobutanol, thimerosal, phenylmercuric acetate and phenylmercuric nitrate.

Penetration enhancers may, for example, be surface active agents; certain organic solvents, such as dimethylsulfoxide and other sulfoxides, dimethylacetamide and pyrrolidone; certain amides of heterocyclic amines, glycols (e.g. propylene glycol); propylene carbonate; oleic acid; alkyl amines and derivatives; various cationic, anionic, nonionic, and amphoteric surface active agents; and the like.

Tonicity adjustors may be added as needed or convenient. They include, but are not limited to, salts, particularly sodium chloride, potassium chloride, mannitol and glycerin, or any other suitable opthalmically acceptable tonicity adjustor.

Various buffers and means for adjusting pH may be used so long as the resulting preparation is ophthalmically acceptable. Accordingly, buffers include acetate buffers, citrate buffers, phosphate buffers and borate buffers for ophthalmic use.

In a similar vein, an ophthalmically acceptable antioxidant for use in the present invention includes, but is not limited to, sodium metabisulfite, sodium thiosulfate, acetylcysteine, butylated hydroxyanisole and butylated hydroxytoluene.

Other excipient components which may be included in the ophthalmic preparations are chelating agents. The preferred chelating agent is edetate disodium, although other chelating agents may also be used in place or in conjunction with it.

In addition to topical therapy, other routes of administration such as oral, parenteral, nasal inhalation, and intrarectal are also contemplated. For these uses, additional conventional pharmaceutical preparations such as tablets, granules, powders, capsules, and sprays may be preferentially required. In such formulations further conventional additives such as binding agents, wetting agents, propellants, lubricants, and stabilizers may also be required.

The route of administration, dosage form, and the effective amount vary according to the potency of the selected PAF antagonist, its physicochemical characteristics, and according to the condition to be treated. The selection of proper dosage is well within the skill of an ordinary skilled physician. Topical formulations are usually administered up to four-times a day. A typical dosage of opthalmic solutions is 1-2 drops in the afflicted eye up to four-times a day.

The use of PAF antagonists as anti-pruritics is advantageous in that they relieve itching by a mechanism independent of histaminergic compounds. Thus, they may be effective in itching diseases which are refractory to antihistamine therapy and may be combined with H₁-antihistamines to provide superior therapy via additive or synergistic interaction.

A more complete appreciation of the invention may be obtained from the following Examples. As a means of providing an atraumatic experimental model of itching, the conjunctiva was used as a convenient tissue site. Pruritogenic agents may be administered to the conjunctiva without the need to traumatize the tissue by injection or scarification. The itch sensation is elicited peripherically by local, atraumatic application of the pruritogen. Of equal importance is the ability of this model to identify locally acting anti-pruritic agents without concerns regarding local tissue trauma or, in the case of systemically administered agents, sedation.

### Example 1

The pruritogenic activity of numerous and structurally diverse autocoids was examined as follows. A 20 µl drop of a solution of the particular autocoid under evaluation was topically administered to one albino guinea-pig eye, the contralateral eye received 20 µl of vehicle as a control. For PAF studies, the R=15 version of formula I was used and was taken up in 0.5% ultrapure bovine serum albumin. The guinea pig was then replaced in its cage and the number of scratching episodes was recorded over the 15 subsequent minute period. The retention of the experimental animal in familiar surroundings is an important factor in experimental design. Scratching, which is the typical mammalian behavioral response to the itch sensation, provides an indication of the intensity of the perceived itch sensation and can be quantified by recording the frequency at which itch-scratch episodes occur per unit time: 15 minutes in the case of these examples. The data obtained on several autocoids at physiological dose levels is summarized in Table 1.

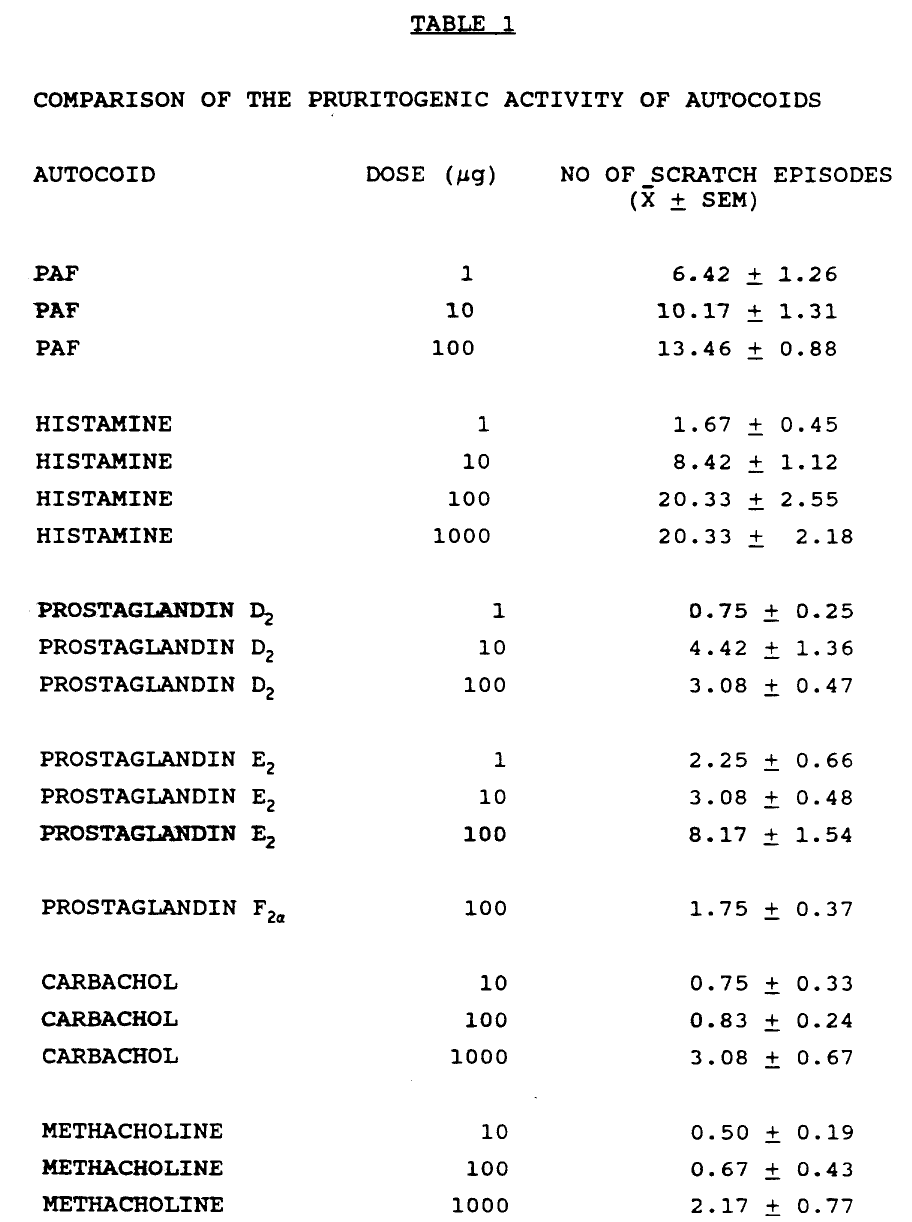

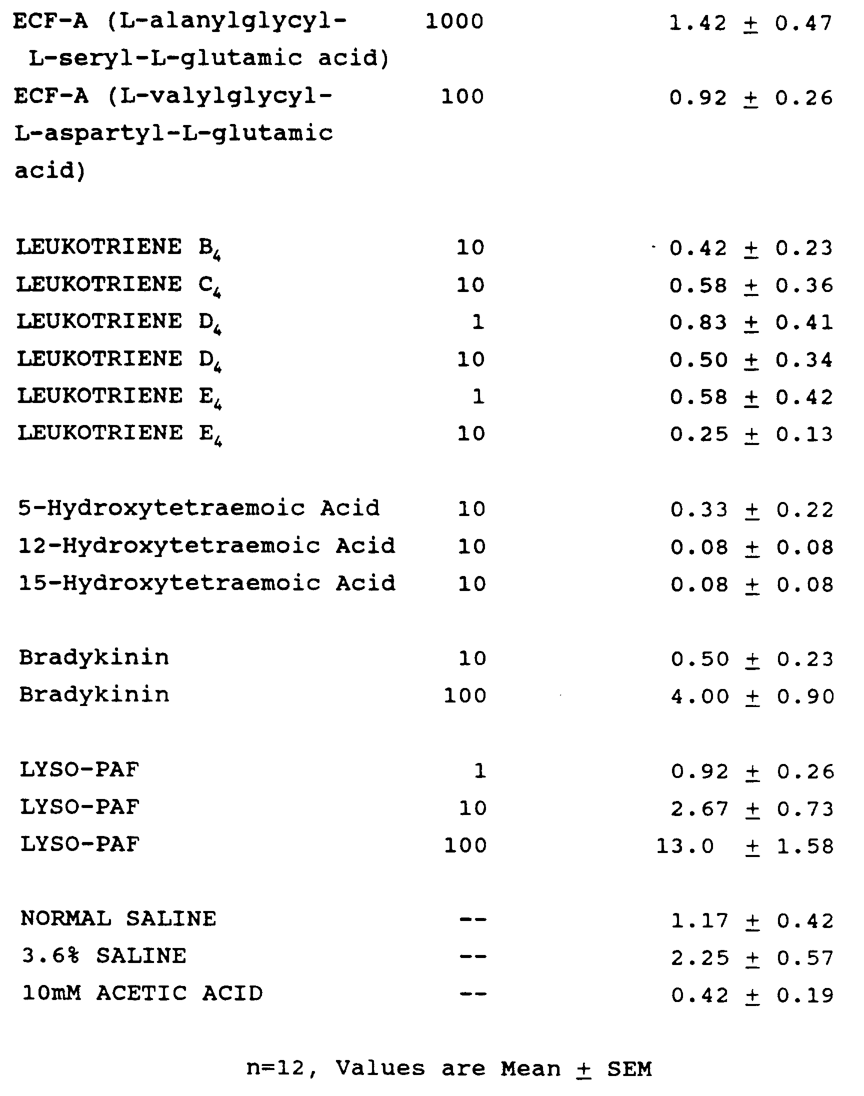

It is apparent from Table 1 that PAF is the most potent pruritogenic agent. The relatively weak activity of lyso-PAF is consistent with a receptor mediated effect. The virtual absence of an itch-scratch response to pain producing stimuli such as hypertonic saline and 10 mM acetic acid provides further validation of the model.

### Example 2

The ability of a 30 minute topical pretreatment with selected PAF antagonists to attenuate PAF induced pruritus is shown in Table 2.

**TABLE 2**

| TREATMENT REGIMEN | CONTROL GROUP | TREATED GROUP |
|---|---|---|
| Compound of formula (II) (10 mg/ml) vs 1 µg PAF | 6.42 ± 1.13 | 2.67 ± 0.68* |
| Compound of formula (II) (10 mg/ml) vs 10 µg PAF | 10.08 ± 0.82 | 6.50 ± 1.09* |
| Compound of formula (III) (10 mg/ml) vs 10 µg PAF | 14.33 ± 1.46 | 8.42 ± 1.37* |
| Compound of formula (IV) (5 mg/ml) 1 µg PAF | 4.00 ± 0.67 | 0.33 ± 0.19** |
| Compound of formula (IV) (5 mg/ml) vs 10 µg PAF | 11.33 ± 1.53 | 5.25 ± 0.98** |
| Pyrilamine (0.1 mg/ml) vs 1 µg PAF | 4.67 ± 0.82 | 4.17 ± 0.91 |
| Pyrilamine (0.1 mg/ml) vs 10 µg PAF | 13.67 ± 1.70 | 14.25 ± 1.75 |
| Values are mean ± SEM | | |

| | | |
|---|---|---|
| *p < 0. 05; | | |
| ** p <0.01; n=12 | | |

Table 2 demonstrates that PAF induced pruritus may be blocked by PAF antagonists but not by the antihistamine pyrilamine: this indicates that PAF does not evoke a pruritic effect by an indirect mechanism involving histamine.

### Example 3

In addition to pharmacological studies on pruritogenic autocoids, the conjunctiva may be used as a convenient site for modelling diseases where itching is a major symptom. In animals presensitized to a particular antigen, subsequent topical challenge with that antigen results in conjunctival itching. This may be regarded as an experimental model of itching which has general relevance to clinically encountered pruritus. In the studies described herein, chicken ovalbumin was used as an antigenic substance and the ability of PAF antagonist pretreatment to block the itching response was examined (Table 3).

**TABLE 3**

| EFFECT OF PAF ANTAGONISTS ON EXPERIMENTAL ALLERGIC ITCHING | | |
|---|---|---|
| TREATMENT REGIMEN | CONTROL GROUP | TREATED GROUP |
| Compound of formula (II) (1 mg/ml) vs 100 µG antigen | 14.38 ± 2.21 | 7.62 ± 0.96* |
| Compound of formula (III) (10 mg/ml) vs 100 µG antigen | 12.25 ± 1.72 | 6.87 ± 1.61* |
| Compound of formula (IV) (10 mg/ml) vs 10 µG antigen | 11.00 ± 2.50 | 5.00 ± 1.10* |

| | | |
|---|---|---|
| *p < 0.05 n=8-10 | | |

These results demonstrate that PAF is a major mediator of itching diseases and that administration of a PAF antagonist provides an effective method for treating pruritus. In addition, PAF antagonists may also be used in combination with antihistamines or glucocorticoids.

## Claims

1. Use of a platelet activating factor (PAF) antagonist for the manufacture of a medicament for the treatment of pruritis.

2. The use of Claim 1, wherein said PAF antagonist is a synthetic analogue of PAF.

3. The use of Claim 2, wherein said synthetic analogue is a compound of formula (II)

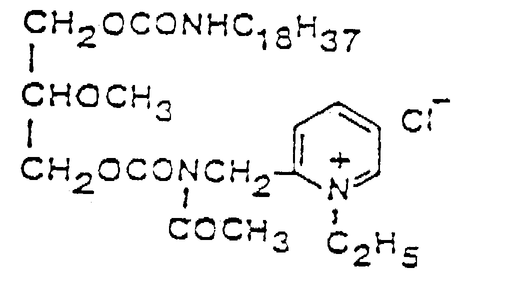

4. The use of Claim 1, wherein said PAF antagonist is a naturally occurring product.

5. The use of Claim 4, wherein said naturally occurring product is a ginkolide or a ginkolide derivative.

6. The use of Claim 4, wherein said naturally occurring product is selected from the group of Ginkgolide A, Ginkolide B, Ginkolide C and Ginkolide M, and derivatives thereof.

7. The use of Claim 6, wherein said naturally occurring product is Ginkgolide B of the formula (III)

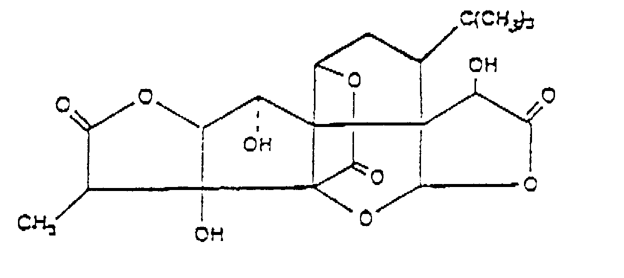

8. The use of Claim 1, wherein said PAF antagonist is a triazolobenzodiazepine.

9. The use of Claim 8, wherein said triazolobenzodiazepine is a compound of the formula (IV)

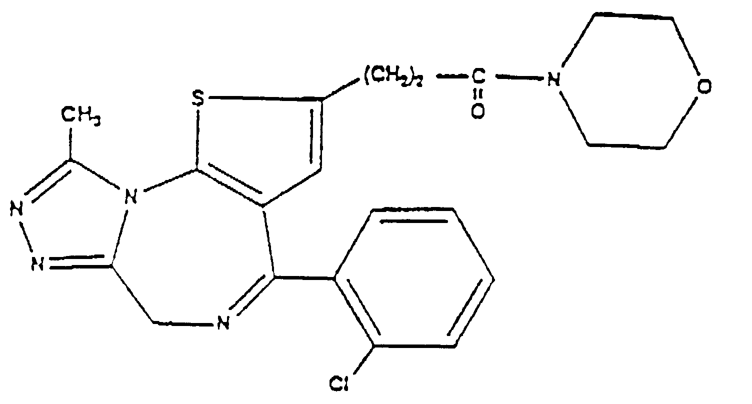

10. The use of any one of Claims 1 to 9, wherein the medicament is in the form of an ophthalmic solution.

11. The use of Claim 10, wherein the ophthalmic solution also comprises at least one further antipruritic agent.

## Patentansprüche

1. Verwendung eines Antagonisten des Blutplättchenaktivierungsfaktors (PAF) für die Herstellung eines Arzneimittels zur Behandlung von Pruritis.

2. Verwendung nach Anspruch 1, wobei der PAF-Antagonist ein synthetisches Analoges von PAF ist.

3. Verwendung nach Anspruch 2, wobei das synthetische Analoge eine Verbindung der Formel (II) ist

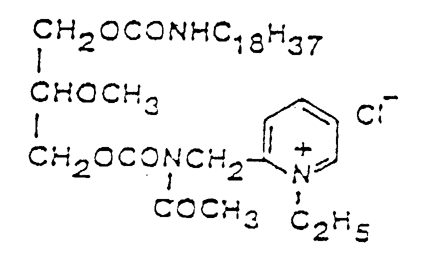

4. Verwendung nach Anspruch 1, wobei der PAF-Antagonist ein natürlich auftretendes Produkt ist.

5. Verwendung nach Anspruch 4, wobei das natürlich auftretende Produkt ein Ginkgolid oder ein Ginkgolid-Derivat ist.

6. Verwendung nach Anspruch 4, wobei das natürlich auftretende Produkt aus der Gruppe Ginkgolid A, Ginkgolid B, Ginkgolid C und Ginkgolid M und Derivaten dieser ausgewählt ist.

7. Verwendung nach Anspruch 6, wobei das natürlich auftretende Produkt Ginkgolid B der Formel (III) ist.

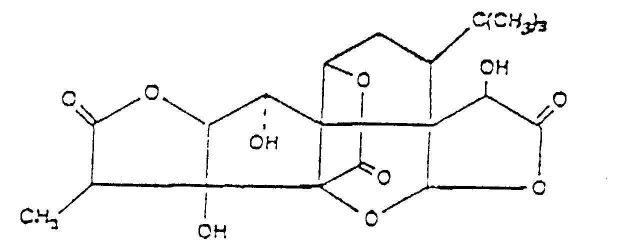

8. Verwendung nach Anspruch 1, wobei der PAF-Antagonist Triazolobenzodiazepin ist.

9. Verwendung nach Anspruch 8, wobei das Triazolobenzodiazepin eine Verbindung der Formel (IV) ist

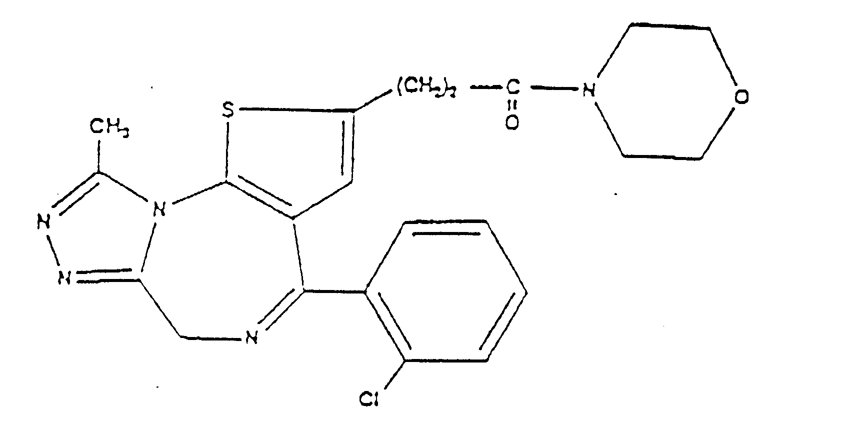

10. Verwendung nach einem der Ansprüche 1 bis 9, wobei das Arzneimittel in der Form einer Lösung für die Augenbehandlung ist.

11. Verwendung nach Anspruch 10, wobei die Lösung für die Augenbehandlung außerdem mindestens ein weiteres Juckreiz verhinderndes Mittel enthält.

## Revendications

1. Emploi d'antagonistes de facteur d'activation plaquettaire (FAP) pour la fabrication d'un médicament destiné au traitement du prurit.

2. Emploi selon la revendication 1, dans lequel ledit antagoniste FAP est un analogue synthétique de FAP.

3. Emploi selon la revendication 2, dans lequel ledit analogue synthétique est un composé de formule (II)

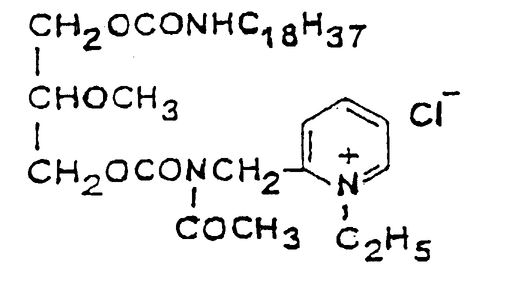

4. Emploi selon la revendication 1, dans lequel ledit antagoniste FAP est un produit qui apparaît spontanément.

5. Emploi selon la revendication 4, dans lequel ledit produit apparaissant spontanément est un ginkolide ou un dérivé de ginkolide.

6. Emploi selon la revendication 4, dans lequel ledit produit apparaissant spontanément est choisi dans le groupe des ginkolide A, ginkolide B, ginkolide C et ginkolide M, et leurs dérivés.

7. Emploi selon la revendication 6, dans lequel ledit produit apparaissant spontanément est le ginkolide B de formule (III)

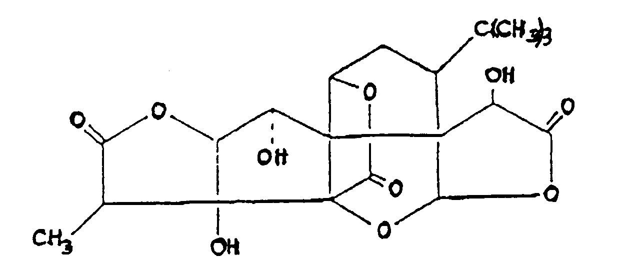

8. Emploi selon la revendication 1, dans lequel ledit antagoniste FAP est une triazolobenzodiazépine.

9. Emploi selon la revendication 8, dans lequel ladite triazolobenzodiazépine est un composé de formule (IV)

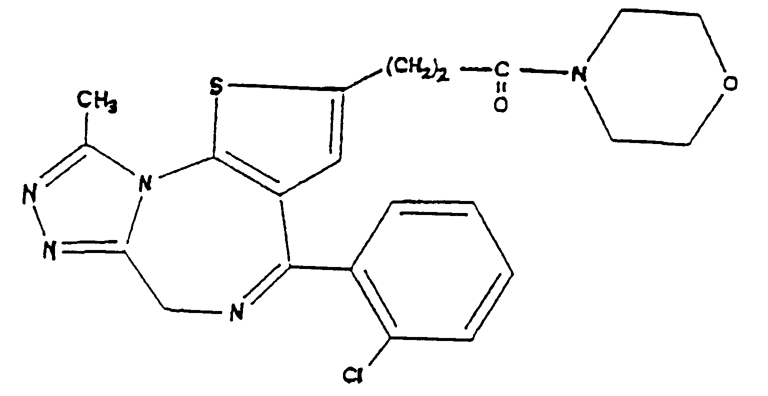

10. Emploi selon l'une quelconque des revendications 1 à 9, dans lequel le médicament a la forme d'une solution ophtalmique.

11. Emploi selon la revendication 10, dans lequel la solution ophtalmique comprend également au moins un autre agent antipruritique.
